# EUROPÄISCHE PATENTSCHRIFT

(11) **EP 3 932 458 B1**
(45) Veröffentlichungstag und Bekanntmachung des Hinweises auf die Patenterteilung: **09.11.2022**
(21) Anmeldenummer: 21166541.9
(22) Anmeldetag: 01.04.2021
(51) Int. Cl.: A61M 16/04, A61M 16/20

(54) **TRACHEOSTOMIEKANÜLE MIT SPRECHVENTIL**
TRACHEOSTOMY CANNULA WITH SPEAKING VALVE
CANULE DE TRACHÉOTOMIE AVEC VALVE DE PHONATION

(30) Priorität: 30.06.2020 DE 102020117223
(43) Veröffentlichungstag der Anmeldung: 05.01.2022
(73) Patentinhaber: Tracoe medical GmbH, 55268 Nieder-Olm (DE)
(72) Erfinder: Schnell, Ralf, 63500 Seligenstadt (DE)
(74) Vertreter: WSL Patentanwälte Partnerschaft mbB

(56) Entgegenhaltungen:
- EP-A1- 1 407 797
- DE-A1- 2 505 123
- DE-A1- 19 543 169
- DE-A1- 19 754 432
- US-A- 4 614 516
- US-A- 4 820 304

## Beschreibung

Die vorliegende Erfindung betrifft eine Tracheostomiekanüle mit einem Sprechventil, wobei das Sprechventil ein Ventilgehäuse aufweist, in dem ein ringförmiger Ventilsitz vorgesehen ist und eine Ventilklappe, die über ein Gelenk oder Scharnier schwenkbar ist, wobei der Ventilsitz und die Ventilklappe so ausgestaltet und angeordnet sind, dass die Ventilklappe in einer ersten Position mit dem Ventilsitz durch Aufliegen so in Kontakt kommt, dass hierdurch das Ventillumen bezüglich einer ersten Strömungsrichtung luftdicht verschlossen ist, und die Ventilklappe in wenigstens einer zweiten Position zwischen der Ventilklappe und dem Ventilsitz eine Öffnung freigibt, durch die ein Luftstrom in eine zu der ersten Strömungsrichtung entgegengesetzte zweite Strömungsrichtung möglich ist.

Zur Stabilisierung des Stomas wird tracheostomierten Patienten eine Tracheostomiekanüle bzw. ein Tracheostomietubus eingesetzt, durch den der Patient ein- und ausatmen kann. Im Zusammenhang mit der vorliegenden Erfindung werden die Begriffe Tracheostomiekanüle und Tracheostomietubus synonym verwendet.

Damit der Patient phonieren bzw. sprechen kann, muss die Kanüle bzw. der Tubus beim Ausatmen kurzzeitig verschlossen werden. Auf diese Weise kann die Luft nicht durch den Hals entweichen, sondern wird über die natürlichen Atemwege geleitet, d.h. am Kehlkopf vorbei durch den Mund oder die Nase nach außen.

Zum vorübergehenden Verschließen der Öffnung der Kanüle werden sogenannte Sprechventile verwendet, welche die Luft beim Einatmen in den Patienten ohne viel Widerstand durchlassen und beim Ausatmen sich schließen, damit die ausgeatmete Luft über die oberen Atemwege geleitet wird.

Es gibt separate Sprechventile, die sich beispielsweise auf den normierten konischen 15 mm Konnektor einer Tracheostomiekanüle aufstecken lassen. Außerdem gibt es auch Kanülen, bei denen das Sprechventil fest an der Außen- oder Innenkanüle montiert ist.

### Würdigung des Standes der Technik für die Ergänzung der Beschreibung*

DE 2 505 123 A1 beschreibt eine Endotrachealkanüle, bei der eine Sprechventileinrichtung so schwenkbar an einer Platte befestigt ist, dass die Sprechventileinrichtung wiederholt und ohne Trennung von der Kanüle aus einer den durchströmbaren Querschnitt der Kanüle praktisch überdeckenden Sprechstellung in eine den durchströmbaren Querschnitt der Kanüle mindestens teilweise freigebende Atmungsstellung gebracht werden kann und umgekehrt.

In DE 195 43 169 A1 wird eine Endotrachealkanüle mit einem Adapterelement zur Befestigung von Innenkanüle und Außenkanüle beschrieben, wobei das Adapterelement einen Ventildeckel aufweist, der in geöffneter Stellung ins Innere der Innenkanüle hineinragt, und wobei die äußere Oberfläche des Ventildeckels in geschlossener Stellung bündig mit der äußeren Oberfläche des Adapterelementes abschließt.

DE 197 54 432 A1 beschreibt ein Sprechventil für Trachealkanülen, mit wenigstens einer flexiblen Ventilzunge am Rohrumfang und/oder an der Seitenwand, wobei die Ventilzunge als Teil eines rohrförmigen Innenkörpers oder als einzeln arretierbare Ventilzunge ausgebildet ist.

Bei dem in EP 1 407 797 A1 beschriebenen Sprechventil ist ein Ventilkörper und eine damit schwenkbar verbundene Ventilklappe vorgesehen, wobei die Ventilklappe an der patientenabgewandten Öffnung angeordnet ist und diese bei Exspiration abdeckt.

In US 4,820,304 A wird ein Tracheostoma-Ventil beschrieben, bei dem die Ebene des ringförmigen Ventilsitzes geneigt ist, um den Widerstand der Ventilanordnung zu verringern, wenn die Ventilscheibe geöffnet wird, wobei die Ventilscheibe einen Laschenabschnitt aufweist, um eine Schwenkbewegung zwischen einer offenen und einer geschlossenen Position zu ermöglichen.

US 4,614,516 A beschreibt ein Ventil mit einem Ventilsitz, der in einem Gehäuse angeordnet ist, und mit einer Ventilmembran, die gelenkig innerhalb des Gehäuses angebracht ist, wobei die Ventilmembran von einer geschlossenen in eine geöffnete Position bewegbar ist, wenn Luft durch die Öffnung vom Tracheostoma zum Ösophagus strömt.

Technisch gibt es verschiedene Lösungen und Bauarten von Ventilen. Bei manchen Ventilen wird eine Membran aus flexiblem Material, wie z.B. Silikon, verwendet, die sich beim Einatmen so verbiegt, dass Luft eingeatmet werden kann. In anderen Fällen werden Ballventile oder Klappenventile eingesetzt.

Klappenventile zeichnen sich dadurch aus, dass eine feste Klappe über ein Scharnier bzw. Gelenk derart am Ventilgehäuse befestigt ist, dass sich die Klappe beim Ausatmen verschließt und beim Einatmen öffnet. Herkömmliche Sprechventile weisen hierfür ein Ventilgehäuse mit einer ringscheibenförmigen bis rohrförmigen Gehäusewand auf, an deren Innenseite ein ringförmiger Ventilsitz vorgesehen ist, auf dessen Auflagefläche die Ventilklappe zum Aufliegen kommt, wenn sie im geschlossenen Zustand ist.

Insbesondere geschwächte Patienten haben manchmal Probleme, beim Einatmen ausreichend Unterdruck zu erzeugen, um die Klappe von Klappenventilen zu öffnen. Oft stellt sich hierdurch Atemnot und in der Folge ein Panikgefühl ein. Es besteht daher ein Bedarf nach einem Sprechventil, dessen Ventilklappe auch durch stark geschwächte Patienten leicht betätigt werden kann.

Diese Aufgabe wird erfindungsgemäß gelöst durch eine Tracheostomiekanüle mit einem Sprechventil, wobei
▪ das Sprechventil ein Ventilgehäuse mit einer ringscheibenförmigen bis rohrförmigen Gehäusewand aufweist,
▪ die Innenseite der Gehäusewand das Ventillumen definiert,
▪ an der Innenseite der Gehäusewand ein ringförmiger Ventilsitz vorgesehen ist, ▪ an der Innenseite der Gehäusewand eine Ventilklappe angeordnet ist,
▪ der Ventilsitz und die Ventilklappe so ausgestaltet und angeordnet sind, dass die Ventilklappe in einer ersten Position mit dem Ventilsitz durch Aufliegen so in Kontakt kommt, dass hierdurch das Ventillumen bezüglich einer ersten Strömungsrichtung luftdicht verschlossen ist, und die Ventilklappe in wenigstens einer zweiten Position zwischen der Ventilklappe und dem Ventilsitz eine Öffnung freigibt, durch die ein Luftstrom in eine zu der ersten Strömungsrichtung entgegengesetzte zweite Strömungsrichtung möglich ist,
wobei der Kontakt zwischen Ventilklappe und Ventilsitz durch eine Auflagefläche auf der einen Seite und eine Auflagekante auf der anderen Seite ausgebildet wird, wobei die Auflagekante eine erste Kantenflanke und eine zweite Kantenflanke aufweist, und wobei der Kontaktwinkel a, den die erste Kantenflanke auf der einen Seite mit der Auflagefläche auf der anderen Seite ausbildet, und der Kontaktwinkel β, den die zweite Kantenflanke auf der einen Seite mit der Auflagefläche auf der anderen Seite ausbildet, jeweils im Bereich von 15° bis 75° liegen.

Die Erfinder haben herausgefunden, dass die Ventilklappe von Sprechventilen unter bestimmten Umständen zu stark am Ventilsitz klebt, z.B. dann, wenn direkt nach dem Reinigen des Sprechventils ein noch nasses Sprechventil eingesetzt wird. Gründe für das Verkleben der Ventilklappe mit dem Ventilsitz können auch an dieser Stelle auftretendes Kondenswasser oder dort eingedrungenes Sekret sein. Die dort dann auftretenden Adhäsions- und Kohäsionskräfte können dazu führen, dass insbesondere geschwächte Patienten nicht mehr den Unterdruck aufbauen können, der zum Öffnen der Klappe erforderlich ist.

Die Erfinder haben daher überlegt, wie das Verkleben der Ventilklappe mit dem Ventilsitz verhindert oder zumindest verringert werden kann. Als beste Lösung hat sich hier herausgestellt, dass der Kontakt zwischen Ventilklappe und Ventilsitz nur auf der einen Seite, d.h. nur auf der Seite der Ventilklappe oder auf der Seite des Ventilsitzes, flächig ausgebildet wird und auf der jeweils gegenüberliegenden Seite durch eine Auflagekante. Entscheidend ist dabei insbesondere der Kontaktwinkel, den die beiden Flanken der Kante mit der Auflagefläche auf der anderen Seite ausbilden.

Im Gegensatz hierzu wird bei herkömmlichen Sprechventilen der Kontakt zwischen Ventilsitz Ventilklappe auf beiden Seiten durch Flächen ausgebildet, die im geschlossenen Zustand flächig aufeinander zum Aufliegen kommen. Zwischen diesen Flächen befindliche Flüssigkeit kann zu Adhäsions- und Kohäsionskräften führen, die bewirken, dass insbesondere geschwächte Patienten nicht mehr den Unterdruck aufbauen können, der zum Öffnen der Ventilklappe erforderlich ist.

Die Erfinder haben herausgefunden, dass der Kontaktwinkel a, den die erste Kantenflanke auf der einen Seite mit der Auflagefläche auf der anderen Seite ausbildet und der Kontaktwinkel β, den die zweite Kantenflanke auf der einen Seite mit der Auflagefläche auf der anderen Seite ausbildet, jeweils im Bereich von 15° bis 75° liegen sollten. In diesem Bereich treten nahezu keine oder nur äußerst geringe Adhäsions- bzw. Kohäsionskräfte auf, wenn zwischen Ventilklappe und Ventilsitz Wasser oder Sekret eindringt. Jedenfalls sind in diesem Bereich die auftretenden Adhäsions- bzw. Kohäsionskräfte so klein, dass sie auch geschwächte Patienten beim Einatmen nicht stören können.

Das Ventilgehäuse des Sprechventils weist eine ringscheibenförmige bis rohrförmige Gehäusewand auf. Diese Gehäusewand besteht aus der nach außen gewandten Außenseite, der nach innen gewandten Innenseite und dem sich dazwischen erstreckenden Material der Gehäusewand. Der Durchmesser des kreisförmigen Querschnitts an der Innenseite der Gehäusewand definiert das Ventillumen, und der Durchmesser des Querschnitts der Außenseite der Gehäusewand definiert den äußeren Umfang des Ventilgehäuses. Die Höhe der Ringscheibe bzw. des Rohres des Ventilgehäuses ermittelt sich anhand der Entfernung zwischen dem proximalen Ende der Ringscheibe bzw. des Rohres und dem distalen Ende der Ringscheibe bzw. des Rohres. Bei bestimmten Ausführungsformen der Erfindung liegt die Höhe der Ringscheibe bzw. des Rohres im Bereich von 2 bis 20 mm. Bei bestimmten Ausführungsformen beträgt die Höhe höchstens 10 mm oder gar nur höchstens 5 mm.

Die Begriffe "distal" und "proximal" werden im Sinne der vorliegenden Erfindung aus der Sicht eines die Tracheostomiekanüle anwendenden Arztes verwendet, d.h. das proximale Ende der Tracheostomiekanüle ist das Ende, welches nach dem Einführen in die Trachea außerhalb des Patientenkörpers verbleibt, während das distale Ende der Tracheostomiekanüle in die Trachea des Patienten eingeführt wird.

Der Ventilsitz ist der Bereich des Ventiles, auf dem die Ventilklappe im geschlossenen Zustand bündig zum Aufliegen kommt, sodass keine Luft mehr durch das Ventilgehäuse strömen kann. Erfindungsgemäß ist der Ventilsitz im Ventilgehäuse angeordnet. Etwas genauer erstreckt sich der Ventilsitz von der Innenseite der Gehäusewand so weit in Richtung der zentralen Längsachse des Ventilgehäuses, dass sich hierdurch ein Auflagebereich ergibt, auf dem die Ventilklappe im geschlossenen Zustand bündig zum Aufliegen kommt.

Bei bestimmten Ausführungsformen ist der Ventilsitz in dem Bereich zwischen der mittleren Höhe der ringscheibenförmigen bis rohrförmigen Gehäusewand und dem proximalen Ende des Ventilgehäuses angeordnet. Bei bevorzugten Ausführungsformen befindet sich der Ventilsitz unmittelbar vor dem proximalen Ende des Ventilgehäuses, sodass die Ventilklappe im geschlossenen Zustand mit dem proximalen Ende des Ventilgehäuses bündig zum Abschluss kommt.

Sofern im Zusammenhang mit der vorliegenden Erfindung auf die Strömungsrichtung Bezug genommen wird, bezeichnet die erste Strömungsrichtung die Richtung, welche die Ausatemluft durch die Tracheostomiekanüle von distal nach proximal nimmt, bis sich das Sprechventil schließt. Die zweite Strömungsrichtung ist die Richtung, welche die Einatemluft durch die Tracheostomiekanüle nimmt, sobald sich das Sprechventil öffnet. Diese Strömungsrichtung verläuft also von proximal nach distal.

Wesentliches Merkmal der vorliegenden Erfindung ist, dass der Kontakt zwischen Ventilklappe und Ventilsitz durch eine Auflagefläche auf der einen Seite und durch eine Auflagekante auf der anderen Seite ausgebildet wird, wobei die jeweiligen Kontaktwinkel in den hier beanspruchten Bereichen liegen müssen. Bei bestimmten Ausführungsformen wird der Kontakt zwischen Ventilklappe und Ventilsitz durch eine Auflagefläche des Ventilsitzes und durch eine Auflagekante der Ventilklappe ausgebildet. Bei alternativen Ausführungsformen wird der Kontakt zwischen Ventilklappe und Ventilsitz durch eine Auflagefläche der Ventilklappe und durch eine Auflagekante des Ventilsitzes ausgebildet.

Bei manchen Ausführungsformen sind der Kontaktwinkel α und der Kontaktwinkel β so zugeordnet, dass der Kontaktwinkel α der Winkel ist, den die Kantenflanke auf der proximalen Seite mit der Auflagefläche ausbildet, und der Kontaktwinkel β der Winkel ist, den die Kantenflanke auf der distalen Seite mit der Auflagefläche ausbildet.

Bei bestimmten Ausführungsformen, bei denen der Kontakt zwischen Ventilklappe und Ventilsitz durch eine Auflagefläche des Ventilsitzes und durch eine Auflagekante der Ventilklappe ausgebildet wird, beträgt der Kontaktwinkel a, den die proximale Kantenflanke mit der Auflagefläche des Ventilsitzes ausbildet, wenigstens 15°, wenigstens 20° oder gar wenigstens 30°. Bei bestimmten Ausführungsformen beträgt der Kontaktwinkel a, den die proximale Kantenflanke mit der Auflagefläche des Ventilsitzes ausbildet, höchstens höchstens 75°, höchstens 70° oder gar nur höchstens 60°.

Die Geometrie der Auflagefläche kann bei verschiedenen Ausführungsformen der Erfindung unterschiedlich gestaltet sein. Bei bestimmten Ausführungsformen ist die Auflagefläche planar. Bei anderen Ausführungsformen ist die Auflagefläche konkav ausgestaltet, und wieder bei anderen Ausführungsformen ist die Auflagefläche konvex ausgestaltet. Im Falle einer konkaven oder konvexen Auflagefläche bestimmt sich der Kontaktwinkel anhand der Tangente an der Kontaktstelle mit der Auflagekante.

Bei bestimmten Ausführungsformen ist die Auflagekante eine scharfe Kante. Bei alternativen Ausführungsformen ist die Auflagekante abgerundet oder angefast. Im Falle einer abgerundeten Auflagekante bestimmt sich der Kontaktwinkel anhand der Tangente an der Kontaktstelle mit der Auflagefläche.

Bei bestimmten Ausführungsformen, bei denen der Kontakt zwischen Ventilklappe und Ventilsitz durch eine Auflagefläche des Ventilsitzes und durch eine Auflagekante der Ventilklappe ausgebildet wird, wird die Auflagefläche durch eine Fase am proximalen Ende der Gehäusewand ausgebildet, wobei die Fase einen Fasenwinkel γ im Bereich von 15° bis 60° aufweist. Bei speziellen Ausführungsformen beträgt der Fasenwinkel γ wenigstens 20° oder gar wenigstens 25°. Bei speziellen Ausführungsformen beträgt der Fasenwinkel γ höchstens 45° oder gar nur höchstens 35°.

Das Ventilgehäuse, der Ventilsitz und/oder die Ventilklappe weisen vorzugsweise eine gewisse Mindeststeifigkeit auf. Bei bestimmten Ausführungsformen besteht das Ventilgehäuse, der Ventilsitz und/oder die Ventilklappe aus einem Material, das ein Elastizitätsmodul bzw. Zug-Elastizitätsmodul im Bereich von 2-400 GPa aufweist.

Bei bestimmten Ausführungsformen besteht das Ventilgehäuse, der Ventilsitz und/oder die Ventilklappe aus Metall mit einem Elastizitätsmodul im Bereich von 40-400 GPa. Bei bestimmten Ausführungsformen ist das Metall ausgewählt unter Silber, einer Silberlegierung oder Edelstahl.

Bei den Ausführungsformen, bei denen das Ventilgehäuse, der Ventilsitz und/oder die Ventilklappe aus Kunststoff besteht, weist dieser vorzugsweise ein Zug-Elastizitätsmodul nach ISO 527 im Bereich zwischen 2,3 und 3,2 GPa auf. Bei diesen Ausführungsformen kann der Kunststoff vorzugsweise ausgewählt sein unter Polystyrol, Polymethylmethacrylat, Polycarbonat, Acrylnitril-Butadien-Styrol-Copolymer (ABS), Polyamid und Polysulfon.

Bei alternativen Ausführungsformen der Erfindung kann das Ventilgehäuse, der Ventilsitz und/oder die Ventilklappe auch aus einer Keramik bestehen, die vorzugsweise die oben näher definierte Steifigkeit aufweist.

Bei bestimmten Ausführungsformen ist die Ventilklappe über ein Gelenk oder Scharnier mit der Gehäusewand schwenkbar verbunden. Bei alternativen Ausführungsformen ist die Ventilklappe nicht fest mit der Gehäusewand verbunden, sondern liegt im geschlossenen Zustand auf dem Ventilsitz auf und schlägt im geöffneten Zustand an einer Anschlagfläche an, die gegenüber der Auflagefläche des Ventilsitzes an der Gehäuseinnenwand vorgesehen ist.

Erfindungsgemäß ist das Sprechventil an einer Tracheostomiekanüle angeordnet. Bei bestimmten Ausführungsformen weist diese Tracheostomiekanüle eine Außenkanüle, eine Innenkanüle und wahlweise einen Konnektor auf, wobei das Sprechventil entweder in oder an der Außenkanüle, der Innenkanüle oder dem Konnektor montiert ist.

Das Sprechventil kann entweder bereits als integraler Bestandteil der Tracheostomiekanüle vorgesehen sein. Alternativ kann das Sprechventil auch als nachrüstbare oder austauschbare Komponente bereitgestellt werden. Dementsprechend wird von der vorliegenden Erfindung auch ein Verfahren zur Herstellung einer Tracheostomiekanüle gemäss Anspruch 10 umfasst.

Für Zwecke der ursprünglichen Offenbarung wird darauf hingewiesen, dass sämtliche Merkmale, wie sie sich aus der vorliegenden Beschreibung, den anhängenden Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen wird hier nur der Kürze und der Lesbarkeit der Beschreibung wegen verzichtet.

In den anhängenden Figuren und der dazugehörigen Figurenbeschreibung sind einzelne konkrete Ausführungsformen der Erfindung schematisch dargestellt. Diese konkreten Ausführungsformen sind lediglich ein Beispiel für mögliche Merkmalskombinationen. Die Erfindung ist jedoch keinesfalls auf diese konkreten Ausführungsformen beschränkt, sondern umfasst alle vom Schutzumfang der Patentansprüche abgedeckten Ausführungsformen, auch wenn sie nicht explizit dargestellt oder beschrieben sind.
- Figur 1:: Stark schematisch dargestellt ist eine Tracheostomiekanüle mit einem im gekennzeichneten Bereich vorgesehenen Sprechventil.
- Figur 2:: Die Darstellung unter Buchstabe a) zeigt ein herkömmliches Sprechventil gemäß Stand der Technik, und die Darstellung unter Buchstabe b) zeigt ein für die vorliegende Erfindung angemessenes Sprechventil im leicht geöffneten Zustand. Die beiden Darstellungen sind stark schematisch vereinfacht.
- Figur 3:: Die Darstellung unter Buchstabe a) zeigt einen Querschnitt durch ein herkömmliches Sprechventil gemäß Stand der Technik, wie es in Figur 2a) gezeigt ist. Die Darstellung unter Buchstabe b) zeigt einen Querschnitt durch ein für die vorliegende Erfindung angemessenes Sprechventil, wie es in Figur 2b) dargestellt ist, im geschlossenen Zustand. Die beiden Darstellungen sind stark schematisch vereinfacht.
- Figur 4:: In Figur 4 ist ein Bereich eines für die vorliegende Erfindung angemessenen Sprechventils im Querschnitt dargestellt, um die zwischen den Kantenflanken der Ventilklappe und der Auflagefläche gebildeten Kontaktwinkel und den Fasenwinkel zu veranschaulichen.

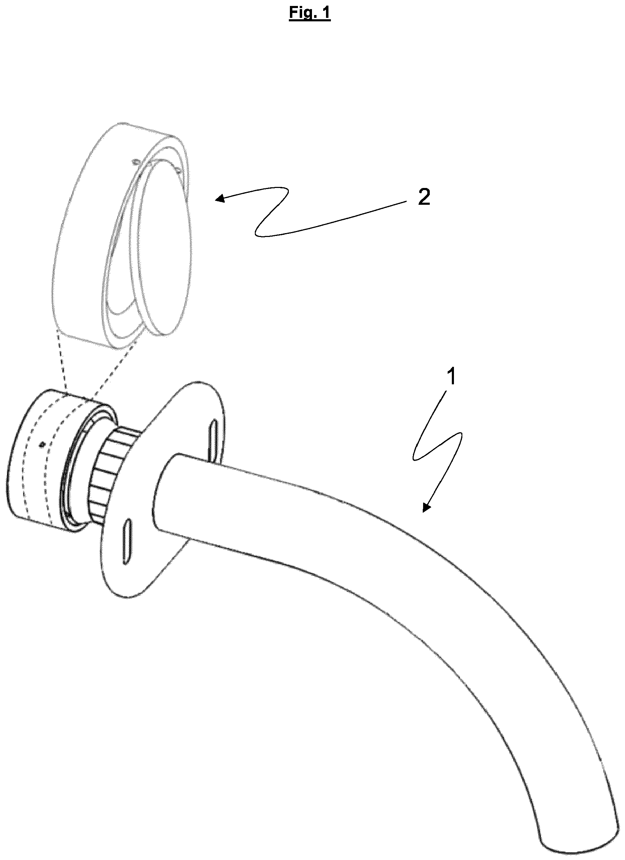

In Figur 1 ist eine erfindungsgemäße Tracheostomiekanüle 1 schematisch dargestellt, in die an deren proximalem Ende ein Sprechventil 2 eingebaut ist. Das Sprechventil befindet sich ir dem mit gestrichelten Linien dargestellten Bereich und ist über der Darstellung der Tracheostomiekanüle noch einmal vergrößert abgebildet.

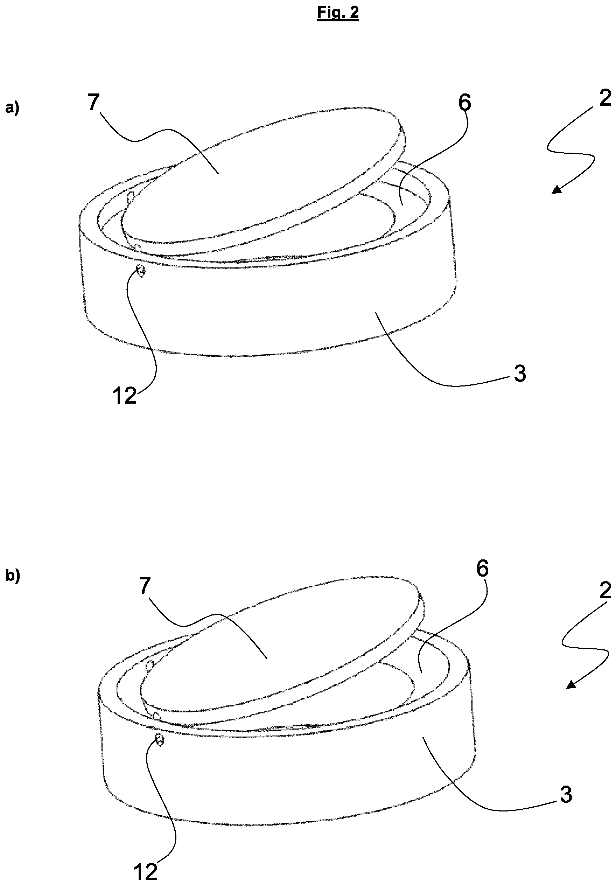

In Figur 2a) ist ein herkömmliches Sprechventil 2 gemäß Stand der Technik dargestellt, dessen Ventilgehäuse 3 eine ringscheibenförmige bis rohrförmige Gehäusewand aufweist, deren Innenseite das Ventillumen definiert. Außerdem ist an der Innenseite der Gehäusewand ein ringförmiger Ventilsitz 6 vorgesehen, auf dessen Auflagefläche die Ventilklappe 7 flächig zum Aufliegen kommt, wenn sie im geschlossenen Zustand ist. Die Ventilklappe 7 ist über ein Scharnier 12 mit dem Ventilgehäuse 3 schwenkbar verbunden und kann so zwischen einer teilweise oder vollständig geöffneten Position und der geschlossenen Position geschwenkt werden.

In Figur 2b) ist ein für die vorliegende Erfindung angemessenes Sprechventil 2 dargestellt, in dessen Ventilgehäuse 3 ebenfalls eine Ventilklappe 7 schwenkbar angeordnet ist. Bei dieser Ausführungsform ist die Wand des Ventilgehäuses so angefast, dass sich hierdurch ein entsprechend abgeschrägter Ventilsitz 6 ergibt. Dadurch kommt die Ventilklappe 7 nur mit deren proximaler Klappenkante mit der vom Ventilsitz 6 gebildeten schrägen Auflagefläche abdichtend in Kontakt.

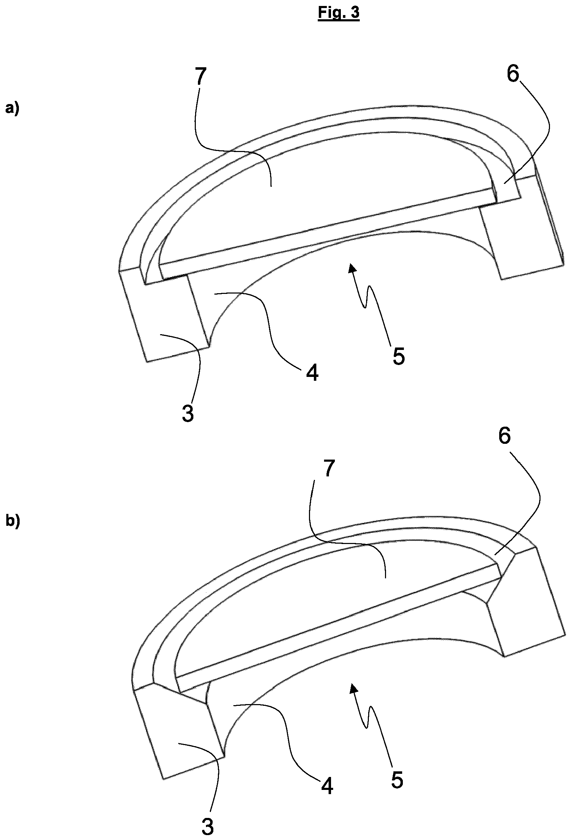

In Figur 3 ist das herkömmliche Sprechventil von Figur 2a) und das Sprechventil von Figur 2b) noch einmal im Querschnitt dargestellt. Gezeigt ist hier jeweils der geschlossene Zustand, sodass gut erkennbar ist, dass beim herkömmlichen Sprechventil die proximale Seite der Ventilklappe 7 auf dem Ventilsitz 6 flächig zum Aufliegen kommt, während beim erfindungsgemäßen Sprechventil nur die proximale Kante der Ventilklappe 7 mit der Auflagefläche des Ventilsitzes 6 abdichtend in Kontakt tritt. In beiden Fällen kann im geschlossenen Zustand keine Ausatemluft in proximaler Richtung durch das Ventillumen 5, das durch die Innenseite 4 der Gehäusewand des Ventilgehäuses 3 definiert wird, strömen.

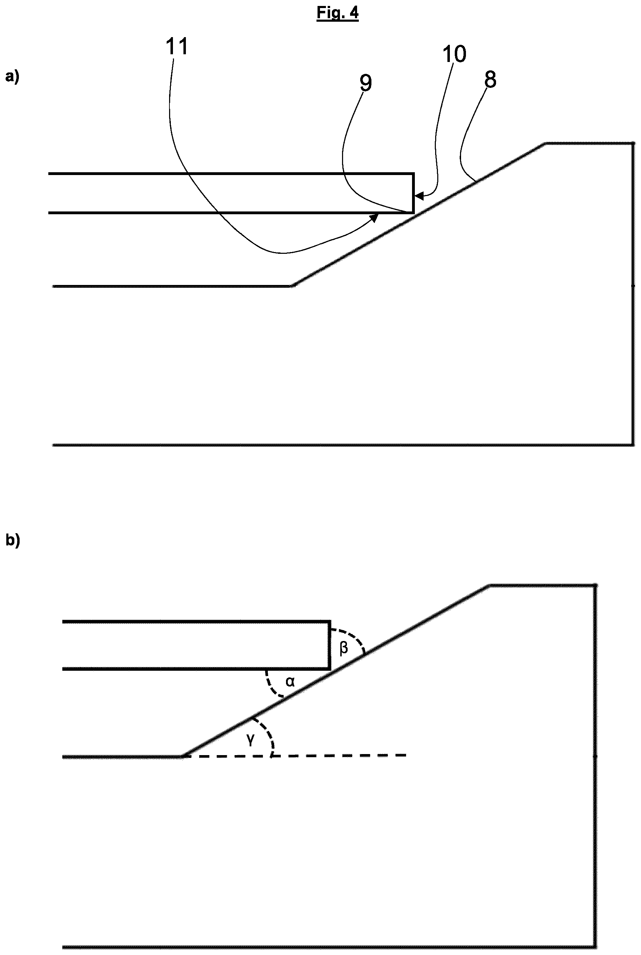

In den Figuren 4a) und b) ist ein Ausschnitt des Sprechventils gemäß Figuren 2b) und 3b) stark schematisiert im Querschnitt dargestellt. Dies soll zur Veranschaulichung des bündigen Kontaktes dienen, der sich im geschlossenen Zustand zwischen der Auflagekante 9 der Ventilklappe und der Auflagefläche 8 am Ventilsitz ausbildet. Des Weiteren sind in dieser Figur die proximale Kantenflanke 11 und die distale Kantenflanke 10 gekennzeichnet. Die proximale Kantenflanke 11 definiert im geschlossenen Zustand mit der Auflagefläche 8 des Ventilsitzes den Kontaktwinkel a, der in Figur 4b) eingezeichnet ist (hier: a = 30°). Die distale Kantenflanke 10 definiert im geschlossenen Zustand zusammen mit der Auflagefläche 8 des Ventilsitzes den Kontaktwinkel β (hier: β = 60°). Die schräge Auflagefläche 8 definiert den Fasenwinkel γ (hier: γ = 30°).

### Bezugszeichenliste

- 1: Tracheostomiekanüle
- 2: Sprechventil
- 3: Ventilgehäuse
- 4: Innenseite der Gehäusewand
- 5: Ventillumen
- 6: Ventilsitz
- 7: Ventilklappe
- 8: Auflagefläche
- 9: Auflagekante
- 10: erste Kantenflanke
- 11: zweite Kantenflanke
- 12: Scharnier

## Patentansprüche

1. Tracheostomiekanüle (1) mit einem Sprechventil (2), wobei
▪ das Sprechventil (2) ein Ventilgehäuse (3) mit einer ringscheibenförmigen bis rohrförmigen Gehäusewand aufweist,
▪ die Innenseite (4) der Gehäusewand das Ventillumen (5) definiert,
▪ an der Innenseite (4) der Gehäusewand ein ringförmiger Ventilsitz (6) vorgesehen ist,
▪ an der Innenseite (4) der Gehäusewand eine Ventilklappe (7) angeordnet ist,
▪ der Ventilsitz (6) und die Ventilklappe (7) so ausgestaltet und angeordnet sind, dass die Ventilklappe (7) in einer ersten Position mit dem Ventilsitz (6) durch Aufliegen so in Kontakt kommt, dass hierdurch das Ventillumen (5) bezüglich einer ersten Strömungsrichtung luftdicht verschlossen ist, und die Ventilklappe (7) in wenigstens einer zweiten Position zwischen der Ventilklappe (7) und dem Ventilsitz (6) eine Öffnung freigibt, durch die ein Luftstrom in eine zu der ersten Strömungsrichtung entgegengesetzte zweite Strömungsrichtung möglich ist,
**dadurch gekennzeichnet, dass**
der Kontakt zwischen Ventilklappe (7) und Ventilsitz (6) durch eine Auflagefläche (8) auf der einen Seite und eine Auflagekante (9) auf der anderen Seite ausgebildet wird, wobei die Auflagekante (9) eine erste Kantenflanke (10) und eine zweite Kantenflanke (11) aufweist, und wobei der Kontaktwinkel a, den die erste Kantenflanke (10) auf der einen Seite mit der Auflagefläche (8) auf der anderen Seite ausbildet, und der Kontaktwinkel β, den die zweite Kantenflanke (11) auf der einen Seite mit der Auflagefläche (8) auf der anderen Seite ausbildet, jeweils im Bereich von 15° bis 75° liegen.

2. Tracheostomiekanüle (1) gemäß Anspruch 1, **dadurch gekennzeichnet, dass** der Kontakt zwischen Ventilklappe (7) und Ventilsitz (6) durch eine Auflagefläche (8) des Ventilsitzes (6) und durch eine Auflagekante (9) der Ventilklappe (7) ausgebildet wird.

3. Tracheostomiekanüle (1) gemäß einem der vorangehenden Ansprüche, **dadurch gekennzeichnet, dass** der Kontakt zwischen Ventilklappe (7) und Ventilsitz (6) durch eine Auflagefläche (8) des Ventilsitzes (6) und durch eine Auflagekante (9) der Ventilklappe (7) ausgebildet wird, wobei der Kontaktwinkel a, der Kontaktwinkel ist, den die proximale Kantenflanke (11) mit der Auflagefläche (8) des Ventilsitzes (6) ausbildet, und wobei der Kontaktwinkel α wenigstens 20° oder gar wenigstens 30° beträgt und höchstens 70° oder gar nur höchstens 60°.

4. Tracheostomiekanüle (1) gemäß einem der vorangehenden Ansprüche, **dadurch gekennzeichnet, dass** die Auflagefläche (8) planar, konkav oder konvex ist.

5. Tracheostomiekanüle (1) gemäß einem der vorangehenden Ansprüche, **dadurch gekennzeichnet, dass** die Auflagekante (9) abgerundet oder angefast ist.

6. Tracheostomiekanüle (1) gemäß einem der vorangehenden Ansprüche, **dadurch gekennzeichnet, dass** das Ventilgehäuse (3), der Ventilsitz (6) und/oder die Ventilklappe (7) aus einem Material besteht, das ein Elastizitätsmodul im Breich von 2 bis 400 GPa aufweist.

7. Tracheostomiekanüle (1) gemäß einem der vorangehenden Ansprüche, **dadurch gekennzeichnet, dass** das Ventilgehäuse (3), der Ventilsitz (6) und/oder die Ventilklappe (7) aus Silber, Edelstahl, Keramik oder Kunststoff bestehen.

8. Tracheostomiekanüle (1) gemäß einem der vorangehenden Ansprüche, **dadurch gekennzeichnet, dass** die Ventiklappe (7) über ein Gelenk oder Scharnier (12) mit der Gehäusewand schwenkbar verbunden ist.

9. Tracheostomiekanüle (1) gemäß einem der vorangehenden Ansprüche, **dadurch gekennzeichnet, dass** die Tracheostomiekanüle (1) eine Außenkanüle, eine Innenkanüle und wahlweise einen Konnektor aufweist, und wobei das Sprechventil entweder in oder an der Außenkanüle, der Innenkanüle oder dem Konnektor montiert ist.

10. Verfahren zur Herstellung einer Tracheostomiekanüle (1) gemäß einem der vorangehenden Ansprüche, wobei man an der Tracheostomiekanüle (1) ein Sprechventil montiert, wobei
▪ das Sprechventil ein Ventilgehäuse (3) mit einer ringscheibenförmigen bis rohrförmigen Gehäusewand aufweist,
▪ die Innenseite (4) der Gehäusewand das Ventillumen (5) definiert,
▪ an der Innenseite (4) der Gehäusewand ein ringförmiger Ventilsitz (6) vorgesehen ist,
▪ an der Innenseite (4) der Gehäusewand eine Ventilklappe (7) angeordnet ist,
▪ der Ventilsitz (6) und die Ventilklappe (7) so ausgestaltet und angeordnet sind, dass die Ventilklappe (7) in einer ersten Position mit dem Ventilsitz (6) durch Aufliegen so in Kontakt kommt, dass hierdurch das Ventillumen (5) bezüglich einer ersten Strömungsrichtung luftdicht verschlossen ist, und die Ventilklappe (7) in wenigstens einer zweiten Position zwischen der Ventilklappe (7) und dem Ventilsitz eine Öffnung freigibt, durch die ein Luftstrom in eine zu der ersten Strömungsrichtung entgegengesetzte zweite Strömungsrichtung möglich ist,
**dadurch gekennzeichnet, dass**
der Kontakt zwischen Ventilklappe (7) und Ventilsitz (6) durch eine Auflagefläche (8) auf der einen Seite und eine Auflagekante (9) auf der anderen Seite ausgebildet wird, wobei die Auflagekante (9) eine erste Kantenflanke (10) und eine zweite Kantenflanke (11) aufweist, und wobei der Kontaktwinkel a, den die erste Kantenflanke (10) auf der einen Seite mit der Auflagefläche (8) auf der anderen Seite ausbildet, und der Kontaktwinkel β, den die zweite Kantenflanke (11) auf der einen Seite mit der Auflagefläche (8) auf der anderen Seite ausbildet, jeweils im Bereich von 15° bis 75° liegen.

11. Verfahren nach Anspruch 10, wobei die Tracheostomiekanüle (1) eine Außenkanüle, eine Innenkanüle und wahlweise einen Konnektor aufweist, und wobei man das Sprechventil an der Außenkanüle, der Innenkanüle oder dem Konnektor montiert.

## Claims

1. Tracheostomy tube (1) with a speaking valve (2), wherein
▪ the speaking valve (2) has a valve housing (3) with a housing wall having a flat ring shape to a tube shape,
▪ the inner surface (4) of the housing wall defines the valve lumen (5),
▪ a ring-shaped valve seat (6) is provided on the inner surface (4) of the housing wall,
▪ a valve flap (7) is arranged on the inner surface (4) of the housing wall,
▪ the valve seat (6) and the valve flap (7) are designed and arranged such that in a first position the valve flap (7) comes into contact with the valve seat (6) by abutment such that the valve lumen (5) is hereby closed in an air-tight manner with respect to a first flow direction, and in at least one second position the valve flap (7) uncovers an opening, through which an air flow in a second flow direction counter to the first flow direction is possible, between the valve flap (7) and the valve seat (6),
**characterized in that**
the contact between valve flap (7) and valve seat (6) is formed by a bearing surface (8) on one side and a bearing edge (9) on the other side, wherein the bearing edge (9) has a first edge flank (10) and a second edge flank (11), and wherein the contact angle a, which the first edge flank (10) on one side forms with the bearing surface (8) on the other side, and the contact angle β, which the second edge flank (11) on one side forms with the bearing surface (8) on the other side, in each case lie in the range of from 15° to 75°.

2. Tracheostomy tube (1) according to claim 1, **characterized in that** the contact between valve flap (7) and valve seat (6) is formed by a bearing surface (8) of the valve seat (6) and by a bearing edge (9) of the valve flap (7).

3. Tracheostomy tube (1) according to one of the preceding claims, **characterized in that** the contact between valve flap (7) and valve seat (6) is formed by a bearing surface (8) of the valve seat (6) and by a bearing edge (9) of the valve flap (7), wherein the contact angle α is the contact angle which the proximal edge flank (11) forms with the bearing surface (8) of the valve seat (6), and wherein the contact angle α is at least 20° or even at least 30° and at most 70° or even only at most 60°.

4. Tracheostomy tube (1) according to one of the preceding claims, **characterized in that** the bearing surface (8) is planar, concave or convex.

5. Tracheostomy tube (1) according to one of the preceding claims, **characterized in that** the bearing edge (9) is rounded or chamfered.

6. Tracheostomy tube (1) according to one of the preceding claims, **characterized in that** the valve housing (3), the valve seat (6) and/or the valve flap (7) consist of a material which has an elastic modulus in the range of from 2 to 400 GPa.

7. Tracheostomy tube (1) according to one of the preceding claims, **characterized in that** the valve housing (3), the valve seat (6) and/or the valve flap (7) consist of silver, stainless steel, ceramic or plastic.

8. Tracheostomy tube (1) according to one of the preceding claims, **characterized in that** the valve flap (7) is pivotably connected to the housing wall via a joint or hinge (12).

9. Tracheostomy tube (1) according to one of the preceding claims, **characterized in that** the tracheostomy tube (1) has an outer tube, an inner tube and optionally a connector, and wherein the speaking valve is fitted either in or on the outer tube, the inner tube or the connector.

10. Method for producing a tracheostomy tube (1) according to one of the preceding claims, wherein a speaking valve is fitted on the tracheostomy tube (1), wherein
▪ the speaking valve has a valve housing (3) with a housing wall having a flat ring shape to a tube shape,
▪ the inner surface (4) of the housing wall defines the valve lumen (5),
▪ a ring-shaped valve seat (6) is provided on the inner surface (4) of the housing wall,
▪ a valve flap (7) is arranged on the inner surface (4) of the housing wall,
▪ the valve seat (6) and the valve flap (7) are designed and arranged such that in a first position the valve flap (7) comes into contact with the valve seat (6) by abutment such that the valve lumen (5) is hereby closed in an air-tight manner with respect to a first flow direction, and in at least one second position the valve flap (7) uncovers an opening, through which an air flow in a second flow direction counter to the first flow direction is possible, between the valve flap (7) and the valve seat,
**characterized in that**
the contact between valve flap (7) and valve seat (6) is formed by a bearing surface (8) on one side and a bearing edge (9) on the other side, wherein the bearing edge (9) has a first edge flank (10) and a second edge flank (11), and wherein the contact angle a, which the first edge flank (10) on one side forms with the bearing surface (8) on the other side, and the contact angle β, which the second edge flank (11) on one side forms with the bearing surface (8) on the other side, in each case lie in the range of from 15° to 75°.

11. Method according to claim 10, wherein the tracheostomy tube (1) has an outer tube, an inner tube and optionally a connector, and wherein the speaking valve is fitted on the outer tube, the inner tube or the connector.

## Revendications

1. Canule de trachéotomie (1) avec une valve de phonation (2),
- la valve de phonation (2) comprenant un boîtier de valve (3) avec une paroi de boîtier en forme de rondelle à forme de tube,
- la face intérieure (4) de la paroi de boîtier définissant le lumen de valve (5),
- sur la face intérieure (4) de la paroi de boîtier, un siège annulaire de valve (6) étant prévu,
- sur la face intérieure (4) de la paroi de boîtier, un clapet de valve (7) étant prévu,
- le siège de valve (6) et le clapet de valve (7) étant configurés et disposés de façon que, dans une première position, le clapet de valve (7) vienne en contact avec le siège de valve par appui de façon que, par cela, le lumen de valve (5) soit étanche à l'air par rapport à une première direction de flux, et que, dans au moins une deuxième position, le clapet de valve (7) libère une ouverture entre le clapet de valve (7) et le siège de valve (6), par laquelle un flux d'air est possible dans une deuxième direction opposée à la première direction,
**caractérisée en ce que**
le contact entre le clapet de valve (7) et le siège de valve (6) est constitué par une surface d'appui (8) sur un côté et un bord d'appui (9) sur l'autre côté, le bord d'appui (9) comprenant un premier flanc de bord (10) et un deuxième flanc de bord (11) et l'angle de contact α qui est formé par le premier flanc de bord (10) sur un côté avec la surface d'appui (8) sur l'autre côté et l'angle de contact β qui est formé par le deuxième flanc de bord (11) sur un côté avec la surface d'appui (8) sur l'autre côté, sont chacun dans la plage de 15° à 75°.

2. Canule de trachéotomie (1) selon la revendication 1, **caractérisée en ce que** le contact entre le clapet de valve (7) et le siège de valve (6) est constitué par une surface d'appui (8) du siège de valve (6) et par un bord d'appui (9) du clapet de valve (7).

3. Canule de trachéotomie (1) selon l'une des revendications précédentes, **caractérisée en ce que** le contact entre le clapet de valve (7) et le siège de valve (6) est constitué par une surface d'appui (8) du siège de valve (6) et par un bord d'appui (9) du clapet de valve (7), l'angle de contact α étant l'angle de contact qui est formé par le flanc de bord proximal (11) avec la surface d'appui (8) du siège de valve (6), et l'angle de contact α étant d'au moins 20° ou même d'au moins 30° et au maximum de 70° ou même au maximum de 60°.

4. Canule de trachéotomie (1) selon l'une des revendications précédentes, **caractérisée en ce que** la surface d'appui (8) est plane, concave ou convexe.

5. Canule de trachéotomie (1) selon l'une des revendications précédentes, **caractérisée en ce que** le bord d'appui (9) est arrondi ou chanfreiné.

6. Canule de trachéotomie (1) selon l'une des revendications précédentes, **caractérisée en ce que** le boîtier de valve (3), le siège de valve (6) et/ou le clapet de valve (7) sont réalisé en un matériau ayant un module d'élasticité dans la plage de 2 à 400 GPa.

7. Canule de trachéotomie (1) selon l'une des revendications précédentes, **caractérisée en ce que** le boîtier de valve (3), le siège de valve (6) et/ou le clapet de valve (7) sont en argent, en acier inoxydable, en céramique ou en matière plastique.

8. Canule de trachéotomie (1) selon l'une des revendications précédentes, **caractérisée en ce que** le clapet de valve (7) est attaché à la paroi de boîtier de façon pivotante à l'aide d'une articulation ou d'une charnière (12).

9. Canule de trachéotomie (1) selon l'une des revendications précédentes, **caractérisée en ce que** la canule de trachéotomie (1) comprend une canule extérieure, une canule intérieure et, au choix, un connecteur, et la valve de phonation étant montée ou dans ou sur la canule extérieure, la canule intérieure ou le connecteur.

10. Procédé de fabrication d'une canule de trachéotomie (1) selon l'une des revendications précédentes, une valve de phonation étant montée sur la canule de trachéotomie (1),
- la valve de phonation (2) comprenant un boîtier de valve (3) avec une paroi de boîtier en forme de rondelle à forme de tube,
- la face intérieure (4) de la paroi de boîtier définissant le lumen de valve (5),
- sur la face intérieure (4) de la paroi de boîtier, un siège annulaire de valve (6) étant prévu,
- sur la face intérieure (4) de la paroi de boîtier, un clapet de valve (7) étant prévu,
- le siège de valve (6) et le clapet de valve (7) étant configurés et disposés de façon que, dans une première position, le clapet de valve (7) vienne en contact avec le siège de valve par appui de façon que, par cela, le lumen de valve (5) soit étanche à l'air par rapport à une première direction de flux, et que, dans au moins une deuxième position, le clapet de valve (7) libère une ouverture entre le clapet de valve (7) et le siège de valve (6), par laquelle un flux d'air est possible dans une deuxième direction opposée à la première direction,
**caractérisé en ce que**
le contact entre le clapet de valve (7) et le siège de valve (6) est constitué par une surface d'appui (8) sur un côté et un bord d'appui (9) sur l'autre côté, le bord d'appui (9) comprenant un premier flanc de bord (10) et un deuxième flanc de bord (11) et l'angle de contact α qui est formé par le premier flanc de bord (10) sur un côté avec la surface d'appui (8) sur l'autre côté et l'angle de contact β qui est formé par le deuxième flanc de bord (11) sur un côté avec la surface d'appui (8) sur l'autre côté, sont chacun dans la plage de 15° à 75°.

11. Procédé selon la revendication 10, la canule de trachéotomie (1) comprenant une canule extérieure, une canule intérieure et, au choix, un connecteur, et la valve de phonation étant montée ou dans ou sur la canule extérieure, la canule intérieure ou le connecteur.
